(19) Europäisches Patentamt
European Patent Office
Office européen des brevets

(11) **EP 3 943 003 A1**

(12) **EUROPEAN PATENT APPLICATION**
published in accordance with Art. 153(4) EPC

(43) Date of publication:
**26.01.2022   Bulletin 2022/04**

(21) Application number: **20772707.4**

(22) Date of filing: **09.03.2020**

(51) International Patent Classification (IPC):
***A61B 5/05*** *(2021.01)*

(52) Cooperative Patent Classification (CPC):
**A61B 5/05**

(86) International application number:
**PCT/JP2020/010076**

(87) International publication number:
**WO 2020/189384 (24.09.2020 Gazette 2020/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.03.2019   JP 2019051809**
**21.08.2019   JP 2019151181**

(71) Applicant: **Toray Engineering Co., Ltd.**
**Tokyo 103-0028 (JP)**

(72) Inventors:
• **SAKAUE, Yusuke**
  **Otsu-shi, Shiga 5202141 (JP)**
• **INAKA, Chisa**
  **Otsu-shi, Shiga 5202141 (JP)**
• **GOTO, Kazushige**
  **Kusatsu-shi, Shiga 5258577 (JP)**

(74) Representative: **Global IP Europe Patentanwaltskanzlei**
**Pfarrstraße 14**
**80538 München (DE)**

(54) **MUSCLE FATIGUE EVALUATION METHOD AND MUSCLE FATIGUE EVALUATION SYSTEM**

(57)   [OBJECT] To provide a non-invasive muscle fatigue evaluation method and muscle fatigue evaluation system with which muscle fatigue that occurs when a non-constant load is applied to a specific muscle can be accurately evaluated.

[MEANS TO SOLVE THE PROBLEMS] At least two electrodes 10 and 20 are disposed at a predetermined interval on a living body surface, a first voltage $V_1$ generated when a first external resistor Rg1 is connected in parallel between the two electrodes, and a second voltage $V_2$ generated when a second external resistor Rg2 is connected in parallel between the two electrode are measured, a bioimpedance between the two electrodes at a muscle site under the living body surface is calculated based on a voltage ratio $V_1/V_2$ between the first voltage $V_1$ and the second voltage $V_2$, and local muscle fatigue at the muscle site is evaluated based on a change over time in the calculated bioimpedance.

[FIG. 2]

EP 3 943 003 A1

**Description**

[TECHINICXAL FIELD]

The present invention relates to a muscle fatigue evaluation method and a muscle fatigue evaluation system for evaluating fatigue of a specific muscle by measuring the impedance in a living body.

[BACKGROUND ART]

As a method for easily evaluating a muscle fatigue state, a method to measure the impedance in a living body by passing a high-frequency current from the outside into the living body is known (see, Patent Literature 1, for example).
However, this method requires a high-frequency current to flow from the outside into the living body, which poses the risk of an electric shock or the like. In addition, it is difficult to evaluate the fatigue of a specific muscle because the overall impedance in the living body is measured.
By contrast, a method to measure an electromyogram is known as a method for non-invasively evaluating a muscle fatigue state (see Non-Patent Literature 1, for example). This method allows the fatigue of a specific muscle to be evaluated by measuring changes in the amplitude and the center frequency of the electromyogram.

[PRIOR ART DOCUMENT]

[PATENT LITERATURE]

[Patent Literature 1] Japanese Patent Application Publication No. 2004-49789 [NON-PATENT LITERATURE]
[Non-Patent Literature 1] Tomohiro Kizuka, Tadashi Masuda, Tohru Kiryu, and Tsugutake Sadoyama, "Practical Usage of Surface Electromyogram," Tokyo Denki University Press, March 2006, pp. 60-62.

[SUMMARY OF THE INVENTION]

[PROBLEM TO BE SOLVED BY THE INVENTION]

However, since an electromyogram is a record of biological signals obtained by causing a muscle to contract, the amplitude and the center frequency change when the magnitude of the load applied to the muscle is changed. Therefore, muscle fatigue can be evaluated only when a load of a certain magnitude has been applied to the muscle. That is, an electromyogram, by itself, is not an index for evaluating muscle fatigue that occurs continuously in a state in which a non-constant load is applied to the muscle.
The present invention is conceived in the light of this point, and a main object thereof is to provide a non-invasive muscle fatigue evaluation method and muscle fatigue evaluation system with which the muscle fatigue that occurs when a non-constant load is applied to a specific muscle can be evaluated.

[MEANS TO SOLVE THE PROBLEMS]

A muscle fatigue evaluation method according to the present invention comprises disposing at least two electrodes at a predetermined interval on a living body surface, measuring a first voltage $V_1$ generated when a first external resistor is connected in parallel between the two electrodes and a second voltage $V_2$ generated when a second external resistor is connected in parallel between the two electrodes, calculating a bioimpedance between the two electrodes at a muscle site under the living body surface based on a voltage ratio $V_1/V_2$ between the first voltage $V_1$ and the second voltage $V_2$, and evaluating local muscle fatigue at the muscle site based on a change overtime in the calculated bioimpedance.
A muscle fatigue evaluation system according to the present invention comprises at least two electrodes disposed at a predetermined interval on a living body surface, a connection means for switchably connecting a first external resistor and a second external resistor in parallel between the two electrodes, a voltage measurement means for measuring a first voltage $V_1$ generated when the first external resistor is connected in parallel between the two electrodes by the connecting means, and a second voltage $V_2$ generated when the second external resistor is connected in parallel between the two electrodes by the connecting means, and an impedance calculation means for calculating a bioimpedance between the two electrodes at a muscle site under the living body surface based on a voltage ratio $V_1/V_2$ between the first voltage $V_1$ and the second voltage $V_2$, local muscle fatigue at the muscle site being evaluated based on a change over time in the calculated bioimpedance.

[EFFECTS OF THE INVENTION]

The present invention allows muscle fatigue that occurs when a non-constant load is applied to a specific muscle to be continuously and non-invasively evaluated.

[BRIEF DESCRIPTION OF THE DRAWINGS]

FIG. 1 is a diagram schematically showing a muscle fatigue evaluation method according to an embodiment of the present invention;
FIG. 2 is an equivalent circuit diagram in the state shown in FIG. 1;
FIGS. 3(a) to 3(c) are graphs of the results of examining the relation between muscle fatigue and bioimpedance;
FIGS. 4(a) and 4(b) are graphs schematically showing the relation between the change in the amount of water in a muscle and the change in bioimpedance;
FIG. 5 is a graph of the results of examining acute muscle fatigue and chronic muscle fatigue; and
FIG. 6 is a diagram showing an arrangement of the two electrodes.

[EMBODIMENTS TO CARRY OUT THE INVENTION]

Embodiments of the present invention will now be described in detail with reference to the drawings. The present invention is not limited to the following embodiments. Also, suitable changes are possible to the extent that such changes do not deviate from the range in which the effect of the present invention is exhibited.

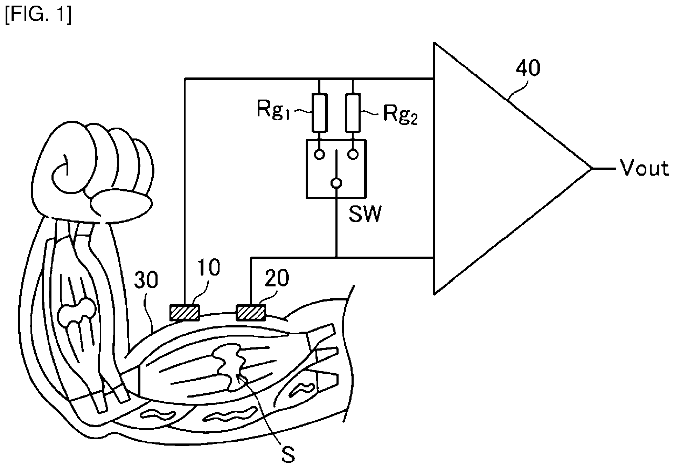

FIG. 1 is a diagram schematically showing a muscle fatigue evaluation method according to an embodiment of the present invention.

As shown in FIG. 1, two electrodes 10 and 20 are disposed at a predetermined interval on a living body surface 30. Here, an example is given in which the two electrodes 10 and 20 are attached to the surface of an upper arm. The voltage generated between the electrodes 10 and 20 is measured by being amplified by an amplifier (voltage measurement means) 40. Also, a first external resistor Rg1 and a second external resistor Rg2 are disposed in parallel between the two electrodes 10 and 20. A switch (connecting means) SW switches between a state in which the first external resistor Rg1 is connected in parallel between the electrodes 10 and 20 and a state in which the second external resistor Rg2 is connected in parallel between the electrodes 10 and 20.

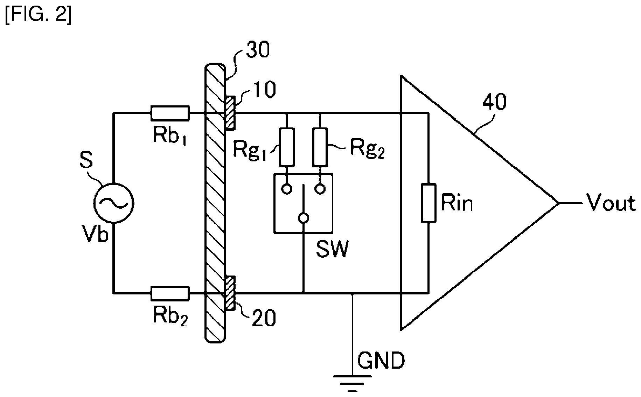

FIG. 2 shows an equivalent circuit diagram of the state shown in FIG. 1.

Here, Vb is the myoelectric potential at the muscle site (brachial muscle) under the living body surface 30 between the two electrodes 10 and 20. This myoelectric potential Vb is generated when the upper arm 30 is exercised, that is, when a load is applied to the brachial muscle.

Also, Rb1 and Rb2 respectively indicate the bioimpedance between a signal source S that generates the myoelectric potential Vb, and the electrodes 10 and 20. The bioimpedance will be described in detail in the description of the relation to muscle fatigue given below. Rin indicates the input resistance of the amplifier 40. The voltage generated between the two electrodes 10 and 20 is amplified by the amplifier 40 and measured as the output voltage Vout.

In the equivalent circuit diagram shown in FIG. 2, the first voltage $V_1$ generated when the first external resistor Rg1 is connected in parallel between the two electrodes 10 and 20 is given by the formula (2).

[First Mathematical Formula]

$$V_1 = \frac{R_{g1}}{R_{b1} + R_{b2} + R_{g1}} V_b \quad \cdot \cdot \cdot (2)$$

The second voltage $V_2$ generated when the second external resistor Rg2 is connected in parallel between the two electrodes 10 and 20 is given by the formula (3).

[Second Mathematical Formula]

$$V_2 = \frac{R_{g2}}{R_{b1} + R_{b2} + R_{g2}} V_b \quad \cdots (3)$$

Therefore, using the formulas (2) and (3), the bioimpedance Zb (= Rb1 + Rb2) between the two electrodes 10 and 20 at the muscle site (brachial muscle) under the living body surface 30 is obtained by the following formula (1).

[Third Mathematical Formula]

$$Z_b = R_{b1} + R_{b2}$$
$$= \frac{R_{g1} \cdot R_{g2}}{R_{g1} - R_{g2} \cdot \frac{V_1}{V_2}} \left( \frac{V_1}{V_2} - 1 \right) \quad \cdots (1)$$

That is, using the formula (1), the bioimpedance Zb between the two electrodes 10 and 20 at the muscle site (brachial muscle) under the living body surface 30 can be calculated based on the voltage ratio ($V_1/V_2$) between the first voltage $V_1$ and the second voltage $V_2$.

On the other hand, it is well known that the blood lactate concentration increases when a load is applied to muscles to the point that muscle fatigue occurs, but it is also known that the water content in muscles also increases. Therefore, when the bioimpedance at the fatigued muscle site is measured, it can be predicted that the bioimpedance will be lower than normal.

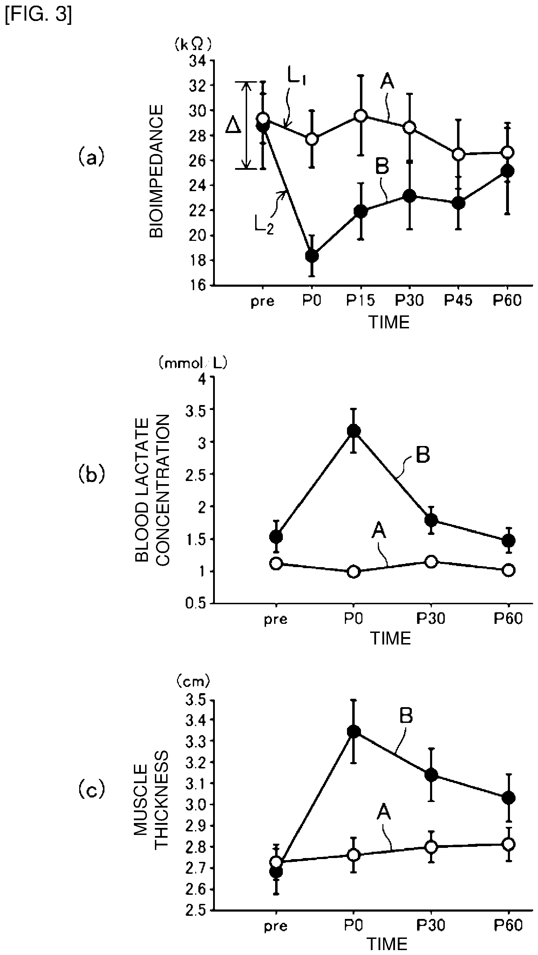

FIGS. 3(a) to 3(c) are graphs of the results of examining the relation between muscle fatigue and bioimpedance. In the test, nine healthy adult men were used as subjects, each of whom used a dumbbell with a weight of 60% of the subject's maximum muscle strength to perform 12 arm curls, and this was repeated for five sets to fatigue the brachial muscle.

FIG. 3(a) is a graph showing the results of measuring the bioimpedance of a subject who performed the above exercise, using the muscle fatigue evaluation method shown in FIGS. 1 and 2. The bioimpedance was measured by attaching two electrodes at an interval of 3.5 cm along the muscle fibers of the brachial muscle in the muscle fatigue evaluation method shown in FIGS. 1 and 2.

Here, the resistance value of the first external resistor Rg1 was set so that the voltage ratio $V_1/V_2$ between the first voltage $V_1$ and the second voltage $V_2$ was about 0.3. The resistance value of the second external resistor Rg2 was set to infinity. Then, the first voltage $V_1$ and the second voltage $V_2$ were measured while using a 4.0-kg dumbbell and holding the elbow joint angle at 90°.

When the resistance value of the second external resistance Rg2 is set to infinity, the above formula (3) is $V_2 \approx Vb$, so the bioimpedance Zb can be obtained by the following formula (4).

[Fourth Mathematical Formula]

$$Z_b = \frac{V_2 - V_1}{V_1} R_{g1} \quad \cdots (4)$$

In FIG. 3(a), the horizontal axis indicates time (minutes) and the vertical axis indicates bioimpedance (kΩ). Graph A in the drawing shows the bioimpedance measured when the above exercise was not performed, and graph B shows the bioimpedance measured when the above exercise was performed. The bioimpedance in the graphs shows the average for nine people.

The bioimpedance at P0 on the horizontal axis indicates the value immediately after the exercise, and the bioimpedance at P15 to P60 indicates the values measured every 15 minutes after the exercise ended.

As shown in FIG. 3(a), the bioimpedance can be seen to decrease greatly immediately after the above exercise (P0). It can also be seen that the bioimpedance gradually returns as time passes (P15 to P60) after the exercise is finished.

FIG. 3(b) is a graph showing the results of measuring the blood lactate concentration by taking blood samples from the subjects at P0, P30, and P60. Graph A in the drawing shows the blood lactate concentration measured when the above exercise was not performed, and graph B shows the blood lactate concentration measured when the exercise was performed. The blood lactate concentration shown in the drawings is the average for nine people.

As shown in FIG. 3(b), it can be seen that the blood lactate concentration is greatly increased immediately after the above exercise (P0). It can also be seen that the blood lactate concentration gradually returns as time passes (P30, P60) after the exercise is finished.

FIG. 3(c) is a graph of the results of measuring the muscle thickness in the upper arm of the subject at P0, P30, and P60. Graph A in the drawing shows the muscle thickness measured when the above exercise was not performed, and graph B shows the muscle thickness measured when exercise was performed. The muscle thickness in the drawings shows the average value for nine people.

As shown in FIG. 3(c), it can be seen that the muscle thickness is greatly increased immediately after the above exercise (P0). It can also be seen that the muscle thickness gradually returns as time passes (P30, P60) after the exercise is finished.

It can be seen from the above results that the change in bioimpedance when a non-constant load is applied to the muscle, such as in biceps curls, is strongly correlated with the change in blood lactate concentration and the change in muscle thickness. That is, the fatigue of a specific muscle can be ascertained as the change in the amount of water in the muscle, and this tells us that the change in bioimpedance can serve as an index reflecting muscle fatigue.

As explained above, when a load to is applied to a muscle and muscle fatigue occurs, the amount of water in the muscle changes, and the fatigue in a specific muscle can be evaluated in real time by ascertaining the change in the amount of water in the muscle as the change in bioimpedance.

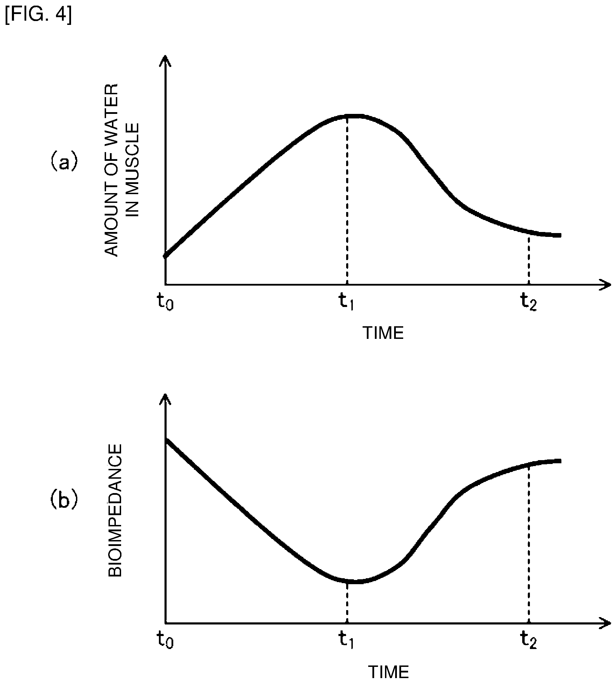

FIG. 4 is a graph schematically showing the relation between the change in the amount of water in a muscle (FIG. 4(a)) and the change in the bioimpedance (FIG. 4(b)). As shown in FIGS. 4(a) and 4(b), when a load to is applied to the muscle and muscle fatigue occurs, the amount of water in the muscle increases (time to to ti), and the bioimpedance decreases along with this (time to to ti). When the load on the muscle is stopped, the amount of water in the muscle returns to its original state (time $t_1$ to $t_2$), and along with this, the bioimpedance also returns to its original state (time $t_1$ to $t_2$). That is, measuring the change over time in the bioimpedance reveals that muscle fatigue accumulates from the time to to $t_1$, and is restored between the times $t_1$ and $t_2$. Consequently, it possible to evaluate in real time the muscle fatigue that occurs when a load is applied to muscles.

As shown in graph A in FIG. 3(a), the bioimpedance may change over time due to factors other than muscle fatigue, even when no exercise is performed. Therefore, when muscle fatigue is evaluated based on the change over time in bioimpedance, it is necessary to distinguish from the change over time due to factors other than muscle fatigue.

Usually, as shown in FIG. 3(a), the amount of change over time (slope) $L_1$ in bioimpedance when exercise is not performed is less than the amount of change over time (slope) $L_2$ in bioimpedance when exercise is performed. Typically, when the bioimpedance immediately after exercise changes by 20% or more with respect to the bioimpedance before exercise, this is believed to be due to muscle fatigue.

On the other hand, when the change over time in the bioimpedance is 20% or less, this is not believed to be due to muscle fatigue. Therefore, muscle fatigue can be correctly evaluated by determining that muscle fatigue has occurred when an amount of the change over time in the bioimpedance calculated after exercise is equal to or greater than a predetermined value.

Also, as shown in FIG. 3(a), a certain fluctuation $\Delta$ occurs in the calculation of the bioimpedance. Accordingly, when muscle fatigue is evaluated based on the change over time in the calculated bioimpedance, it is necessary to distinguish from the change over time due to fluctuation in the bioimpedance. Therefore, the bioimpedance is calculated a number of times before exercise to obtain the fluctuation of the calculated bioimpedance, and muscle fatigue is evaluated based on the change over time when the bioimpedance calculated after exercise is greater than or equal to the fluctuation, which allows muscle fatigue to be correctly evaluated.

On the other hand, it is known that when a load is applied to a muscle, transient muscle fatigue (acute muscle fatigue) initially occurs, after which the muscle fatigue recovers, and then muscle fatigue (chronic muscle fatigue) occurs again.

The muscle fatigue evaluation method of this embodiment allows these acute muscle fatigue and chronic muscle fatigue to be evaluated in real time.

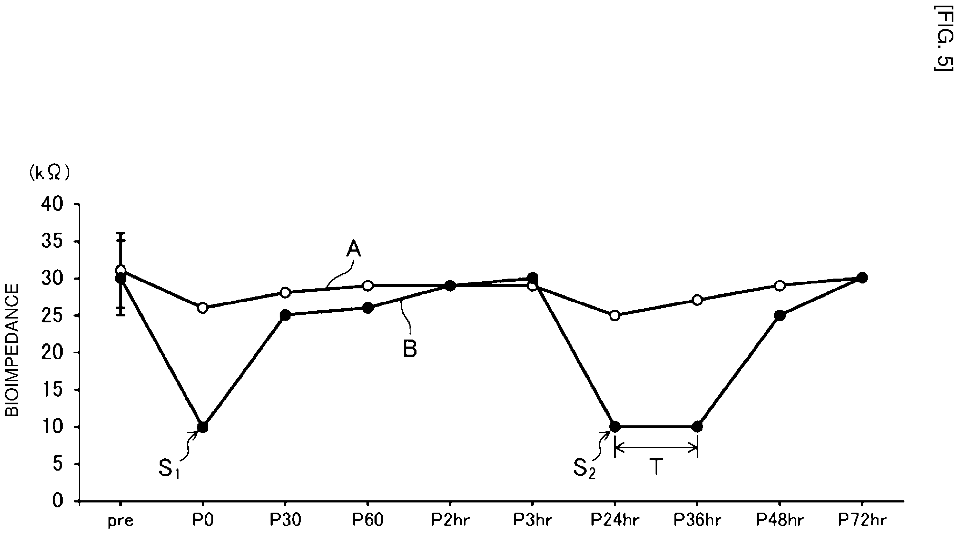

FIG. 5 is a graph of the results of measuring the bioimpedance of a subject who has exercised, using the muscle fatigue evaluation method shown in FIGS. 1 and 2. The bioimpedance was measured by the same method as that shown in FIG. 3(a).

In FIG. 5, the horizontal axis indicates time and the vertical axis indicates bioimpedance (k$\Omega$). Graph A in the drawing shows the bioimpedance measured when exercise was not performed, and graph B shows the bioimpedance measured when exercise was performed.

The bioimpedance at pre on the horizontal axis indicates the value before exercise, and the bioimpedance at P0 on the horizontal axis indicates the value immediately after exercise is finished. P30, P60, P2hr, P3hr, P24hr, P36hr, P48hr, and P72hr on the horizontal axis indicate 30 minutes, 60 minutes, 2 hours, 3 hours, 24 hours, 36 hours, 48 hours, and 72 hours after exercise, respectively.

As shown in FIG. 5, immediately after exercise is finished (P0), the bioimpedance can be seen to have greatly decreased, reaching the minimal value $S_1$. After this, it can be seen that the bioimpedance gradually returns as time passes (P30 to P60), reaching the pre-exercise value about 2 hours later (P2hr).

As more time passes, the bioimpedance again greatly decreases after about 3 hours (P3hr), and can be seen to have reached the second minimal value $S_2$ after about 24 hours (P24hr). The second minimal value $S_2$ continues for about 12 hours (T), and after 36 hours (P36hr), the bioimpedance can be seen to have gradually returned to the pre-exercise value after about 72 hours (P72hr).

In the change over time in the bioimpedance shown in FIG. 5, the muscle fatigue at the point when the initial minimal value $S_1$ is reached can be determined to be acute muscle fatigue. Also, the muscle fatigue at the point when the next minimal value $S_2$ is reached after the initial minimal value $S_1$ can be determined to be chronic muscle fatigue.

Also, in the change over time in the bioimpedance shown in FIG. 5, the duration of the minimal value $S_1$ is the time it takes from the rapid decrease to the rapid increase, whereas the duration of the minimal value $S_2$ is the time it takes from the rapid decrease to the rapid increase after a certain amount of time has elapsed. That is, the duration of the minimal value $S_2$ is longer than the duration of the minimal value $S_1$. Therefore, muscle fatigue when the duration of the minimal value $S_1$ is short can be determined to be acute muscle fatigue, and muscle fatigue when the duration of the minimal value $S_2$ is long can be determined to be chronic muscle fatigue.

Thus, with the muscle fatigue evaluation method in this embodiment, acute muscle fatigue and chronic muscle fatigue that occur after applying a load to a muscle can be evaluated in real time and non-invasively. In particular, evaluating chronic muscle fatigue used to demand a high degree of specialized knowledge, but in this embodiment, chronic muscle fatigue can be evaluated by a simple method. As a result, it is possible to prevent the risk of injury or the like posed by training in a state in which chronic muscle fatigue remains, and to prevent a decrease in the training effect, as well as the occurrence of overtraining due to excessive training.

The muscle fatigue evaluation method in this embodiment comprises disposing at least two electrodes 10 and 20 at a predetermined interval on the living body surface, measuring the first voltage $V_1$ generated when the first external resistor Rg1 is connected in parallel between the two electrodes 10 and 20 and the second voltage $V_2$ generated when the second external resistor Rg2 is connected in parallel between the two electrodes 10 and 20, calculating the bioimpedance Zb between the two electrodes 10 and 20 at a muscle site under the living body surface based on the voltage ratio $V_1/V_2$ between the first voltage $V_1$ and the second voltage $V_2$, and evaluating the local muscle fatigue at the muscle site based on the change over time in the calculated bioimpedance Zb.

This allows muscle fatigue that occurs when a non-constant load is applied to a specific muscle to be evaluated in real time. Also, since muscle fatigue at a specific muscle site to be evaluated for fatigue can be evaluated merely by disposing two electrodes at this site, muscle fatigue can be evaluated accurately and non-invasively.

Because the present invention provides this effect, muscle fatigue can be evaluated for each specific muscle in daily training, for example, so more effective training can be performed. Also, since muscle fatigue can be evaluated in real time, this prevents the exacerbation of a condition or the occurrence of injuries caused by overtraining.

The present invention was described above in terms of a preferred embodiment, but this description is not a limitation, and various modifications are, of course, possible.

For example, in the above embodiment, the two electrodes 10 and 20 are disposed on the living body surface, but a ground electrode may be disposed on the living body surface and the voltage generated between the two electrodes 10 and 20 may be measured with a differential amplifier 40. Here again, the bioimpedance between the two electrodes 10 and 20 can be obtained from the above formula (1). Also, since the first voltage $V_1$ and the second voltage $V_2$ are measured by being amplified by the differential amplifier 40 after taking the difference therebetween, external noise can be removed. This allows the bioimpedance Zb to be measured more accurately.

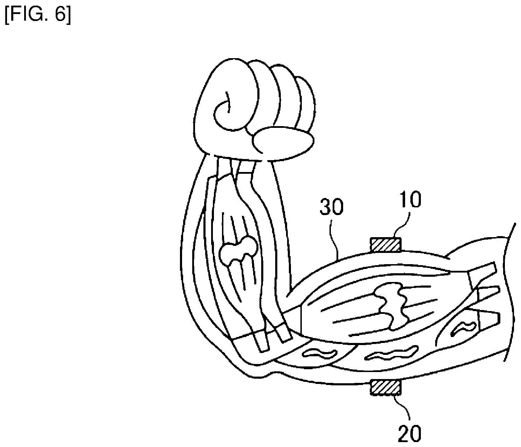

Also, in the above embodiment, when evaluating muscle fatigue at a muscle site formed of muscle fibers (brachial muscle), the two electrodes are disposed near each other along the muscle fibers, but as shown in FIG. 6, they may instead be disposed near each other so as to surround the muscle fibers.

The present invention can also be used as a muscle fatigue evaluation system. That is, the muscle fatigue evaluation system according to the present invention comprises at least two electrodes disposed at a predetermined interval on the living body surface, a connection means for switchably connecting a first external resistor and a second external resistor in parallel between the two electrodes, a voltage measurement means for measuring the first voltage $V_1$ generated when the first external resistor is connected in parallel between the two electrodes by the connecting means, and the second voltage $V_2$ generated when the second external resistor is connected in parallel between the two electrodes by the connecting means, and an impedance calculation means for calculating the bioimpedance between the two electrodes at a muscle site under the living body surface based on the voltage ratio $V_1/V_2$ between the first voltage $V_1$ and the second voltage $V_2$. The local muscle fatigue at the muscle site is then evaluated based on the change over time in the calculated bioimpedance.

[DESCRIPTION OF THE REFERENCE NUMERALS]

| | |
|---|---|
| 10 | electrode |
| 20 | electrode |
| 30 | living body surface |
| 40 | amplifier (differential amplifier) |
| Rg1 | first external resistor |
| Rg2 | second external resistor |

**Claims**

1. A muscle fatigue evaluation method comprising:

   disposing at least two electrodes at a predetermined interval on a living body surface;
   measuring a first voltage $V_1$ generated when a first external resistor is connected in parallel between the two electrodes and a second voltage $V_2$ generated when a second external resistor is connected in parallel between the two electrodes;
   calculating a bioimpedance between the two electrodes at a muscle site under the living body surface based on a voltage ratio $V_1/V_2$ between the first voltage $V_1$ and the second voltage $V_2$; and
   evaluating local muscle fatigue at the muscle site based on a change over time in the calculated bioimpedance.

2. The muscle fatigue evaluation method according to claim 1, wherein
   the bioimpedance Zb is measured based on the following formula (1):
   [First Mathematical Formula]

$$Z_b = \frac{R_{g1} \cdot R_{g2}}{R_{g1} - R_{g2} \cdot \frac{V_1}{V_2}} \left( \frac{V_1}{V_2} - 1 \right) \quad \cdots (1)$$

   where Rg1 represents the first external resistance and Rg2 represents the second external resistance.

3. The muscle fatigue evaluation method according to claim 1 or 2, wherein
   one of the first external resistance and the second external resistance has infinite resistance.

4. The muscle fatigue evaluation method according to any one of claims 1 to 3, wherein
   the two electrodes are disposed near each other on the living body surface at a muscle site formed of muscle fibers along the muscle fibers.

5. The muscle fatigue evaluation method according to any one of claims 1 to 3, wherein
   the two electrodes are disposed near each other on the living body surface at a muscle site formed of muscle fibers so as to surround the muscle fibers.

6. The muscle fatigue evaluation method according to claim 1, wherein

   the bioimpedance between the two electrodes is calculated after exercise, and
   the muscle fatigue is determined to have occurred when an amount of the change over time in the bioimpedance calculated after exercise is equal to or greater than a predetermined value.

7. The muscle fatigue evaluation method according to claim 1, wherein

   the bioimpedance between the two electrodes is calculated before exercise to obtain fluctuation in the calculated bioimpedance, and
the muscle fatigue is evaluated based on the change over time when the bioimpedance calculated after exercise is equal to or greater than the fluctuation.

8. The muscle fatigue evaluation method according to claim 6 or 7, wherein
the muscle fatigue at a point when an initial minimal value is reached in the change over time in the calculated bioimpedance is determined to be acute muscle fatigue, and the muscle fatigue at a point when the next minimal value is reached from the minimal value is determined to be chronic muscle fatigue.

9. The muscle fatigue evaluation method according to claim 6 or 7, wherein
the muscle fatigue at a point when a duration of a minimal value in the change over time in the calculated bioimpedance is short is determined to be acute muscle fatigue, and the muscle fatigue at a point when the duration of the minimal value is long is determined to be chronic muscle fatigue.

10. A muscle fatigue evaluation system comprising:

at least two electrodes disposed at a predetermined interval on a living body surface;
a connection means for switchably connecting a first external resistor and a second external resistor in parallel between the two electrodes;
a voltage measurement means for measuring a first voltage $V_1$ generated when the first external resistor is connected in parallel between the two electrodes by the connecting means, and a second voltage $V_2$ generated when the second external resistor is connected in parallel between the two electrodes by the connecting means; and
an impedance calculation means for calculating a bioimpedance between the two electrodes at a muscle site under the living body surface based on a voltage ratio $V_1/V_2$ between the first voltage $V_1$ and the second voltage $V_2$,
local muscle fatigue at the muscle site being evaluated based on a change over time in the calculated bioimpedance.

[FIG. 1]

[FIG. 2]

[FIG. 3]

[FIG. 4]

(a)

AMOUNT OF WATER IN MUSCLE

$t_0$        $t_1$        $t_2$

TIME

(b)

BIOIMPEDANCE

$t_0$        $t_1$        $t_2$

TIME

[FIG. 5]

[FIG. 6]

**EP 3 943 003 A1**

**INTERNATIONAL SEARCH REPORT**

| | International application No. |
|---|---|
| | PCT/JP2020/010076 |

A. CLASSIFICATION OF SUBJECT MATTER
Int. Cl. A61B5/05(2006.01)i
FI: A61B5/05 B

According to International Patent Classification (IPC) or to both national classification and IPC

B. FIELDS SEARCHED

Minimum documentation searched (classification system followed by classification symbols)
Int. Cl. A61B5/05-5/053

Documentation searched other than minimum documentation to the extent that such documents are included in the fields searched
Published examined utility model applications of Japan    1922-1996
Published unexamined utility model applications of Japan  1971-2020
Registered utility model specifications of Japan          1996-2020
Published registered utility model applications of Japan  1994-2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2004-201877 A (TANITA CORP.) 22 July 2004, entire text, all drawings | 1-10 |
| A | JP 2004-141223 A (TANITA CORP.) 20 May 2004, entire text, all drawings | 1-10 |
| A | JP 2018-27199 A (TORAY ENGINEERING CO., LTD.) 22 February 2018, entire text, all drawings | 1-10 |

☐ Further documents are listed in the continuation of Box C.    ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25.05.2020 | 02.06.2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

15

International application No.

PCT/JP2020/010076

| Patent Documents referred to in the Report | Publication Date | Patent Family | Publication Date |
|---|---|---|---|
| JP 2004-201877 A | 22.07.2004 | US 2004/0092840 A1 entire document EP 1433420 A1 | |
| JP 2004-141223 A | 20.05.2004 | US 2004/0082877 A1 entire document EP 1413250 A1 | |
| JP 2018-27199 A | 22.02.2018 | US 2019/0175046 A1 entire document WO 2018/034058 A1 | |

Form PCT/ISA/210 (patent family annex) (January 2015)

## REFERENCES CITED IN THE DESCRIPTION

**Patent documents cited in the description**

- JP 2004049789 A

**Non-patent literature cited in the description**

- **TOMOHIRO KIZUKA ; TADASHI MASUDA ; TO-HRU KIRYU ; TSUGUTAKE SADOYAMA.** Practical Usage of Surface Electromyogram. Tokyo Denki University Press, March 2006, 60-62